# EUROPEAN PATENT APPLICATION

(11) **EP 3 903 806 A1**
(43) Date of publication of application: **03.11.2021**
(21) Application number: 20914763.6
(22) Date of filing: 22.12.2020
(51) Int. Cl.: A61K 38/18, A61K 48/00, A61P 29/00, A61P 9/12, A61P 9/10, A61P 25/04

(54) **PHARMACEUTICAL COMPOSITION FOR TREATING TRPV1 ACTIVITY-MEDIATED DISEASES**

(30) Priority: 20.01.2020 KR 20200007433
(71) Applicant: Rudacure Corporation, Incheon 21988 (KR)
(72) Inventor: KIM, Yong Ho, Incheon 21988 (KR); KIM, Seung Hoon, Incheon 21988 (KR); JEON, Ha Won, Incheon 21988 (KR); HA, Yu Seung, Incheon- gu 21988 (KR)
(74) Representative: Louis Pöhlau Lohrentz
(86) International application number: PCT/KR2020/018895
(87) International publication number: WO 2021/149925

(57) **Abstract**

The present invention relates to a novel therapeutic pharmaceutical composition, and more specifically, the present invention relates to a pharmaceutical composition for treating TRPV1-mediated diseases.

Since the pharmaceutical composition for treating pain using inhibition of TRPV1 activity of the present invention exhibits an excellent effect of suppressing neuropathic pain caused by spinal nerve damage, it can be used as a novel pain therapeutic agent for various pain conditions and diseases related to the TRPV1 channel such as arthritis and diabetic peripheral neuropathy. In addition, since it effectively inhibits TRPV1 activity, it can be utilized as a therapeutic agent for treating various TRPV1-mediated diseases.

SEQUENCE LISTING

<110>  RUDACURE Corporation

<120>  Pharmaceutical composition for treating TRPV1-mediated diseases

<130>  61923EPW/AW/nk

<141>  22.12.2020

<150>  KR 10-2020-0007433
<151>  2020-01-20

<160>  11

<170>  PatentIn version 3.2

<210>  1
<211>  109
<212>  PRT
<213>  Artificial

<220>
<223>  GDF-11 mature peptide

<400>  1

Asn Leu Gly Leu Asp Cys Asp Glu His Ser Ser Glu Ser Arg Cys Cys
1               5                   10                  15

Arg Tyr Pro Leu Thr Val Asp Phe Glu Ala Phe Gly Trp Asp Trp Ile
            20                  25                  30

Ile Ala Pro Lys Arg Tyr Lys Ala Asn Tyr Cys Ser Gly Gln Cys Glu
        35                  40                  45

Tyr Met Phe Met Gln Lys Tyr Pro His Thr His Leu Val Gln Gln Ala
    50                  55                  60

Asn Pro Arg Gly Ser Ala Gly Pro Cys Cys Thr Pro Thr Lys Met Ser
65                  70                  75                  80

Pro Ile Asn Met Leu Tyr Phe Asn Asp Lys Gln Gln Ile Ile Tyr Gly
                85                  90                  95

Lys Ile Pro Gly Met Val Val Asp Arg Cys Gly Cys Ser
            100                 105

<210>  2
<211>  407
<212>  PRT
<213>  Artificial

<220>
<223>  GDF-11 CDS full sequence

<400>  2

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Ala Gly Val Gly Gly Glu Arg Ser
        35                  40                  45

Ser Arg Pro Ala Pro Ser Val Ala Pro Glu Pro Asp Gly Cys Pro Val
    50                  55                  60

Cys Val Trp Arg Gln His Ser Arg Glu Leu Arg Leu Glu Ser Ile Lys
65                  70                  75                  80

Ser Gln Ile Leu Ser Lys Leu Arg Leu Lys Glu Ala Pro Asn Ile Ser
                85                  90                  95

Arg Glu Val Val Lys Gln Leu Leu Pro Lys Ala Pro Pro Leu Gln Gln
            100                 105                 110

Ile Leu Asp Leu His Asp Phe Gln Gly Asp Ala Leu Gln Pro Glu Asp
        115                 120                 125

Phe Leu Glu Glu Asp Glu Tyr His Ala Thr Thr Glu Thr Val Ile Ser
    130                 135                 140

Met Ala Gln Glu Thr Asp Pro Ala Val Gln Thr Asp Gly Ser Pro Leu
145                 150                 155                 160

Cys Cys His Phe His Phe Ser Pro Lys Val Met Phe Thr Lys Val Leu
                165                 170                 175

Lys Ala Gln Leu Trp Val Tyr Leu Arg Pro Val Pro Arg Pro Ala Thr
            180                 185                 190

Val Tyr Leu Gln Ile Leu Arg Leu Lys Pro Leu Thr Gly Glu Gly Thr
        195                 200                 205

Ala Gly Gly Gly Gly Gly Gly Arg Arg His Ile Arg Ile Arg Ser Leu
    210                 215                 220

Lys Ile Glu Leu His Ser Arg Ser Gly His Trp Gln Ser Ile Asp Phe
225                 230                 235                 240

Lys Gln Val Leu His Ser Trp Phe Arg Gln Pro Gln Ser Asn Trp Gly

245                 250                 255

Ile Glu Ile Asn Ala Phe Asp Pro Ser Gly Thr Asp Leu Ala Val Thr
            260                 265                 270

Ser Leu Gly Pro Gly Ala Glu Gly Leu His Pro Phe Met Glu Leu Arg
        275                 280                 285

Val Leu Glu Asn Thr Lys Arg Ser Arg Arg Asn Leu Gly Leu Asp Cys
    290                 295                 300

Asp Glu His Ser Ser Glu Ser Arg Cys Cys Arg Tyr Pro Leu Thr Val
305                 310                 315                 320

Asp Phe Glu Ala Phe Gly Trp Asp Trp Ile Ile Ala Pro Lys Arg Tyr
                325                 330                 335

Lys Ala Asn Tyr Cys Ser Gly Gln Cys Glu Tyr Met Phe Met Gln Lys
            340                 345                 350

Tyr Pro His Thr His Leu Val Gln Gln Ala Asn Pro Arg Gly Ser Ala
        355                 360                 365

Gly Pro Cys Cys Thr Pro Thr Lys Met Ser Pro Ile Asn Met Leu Tyr
    370                 375                 380

Phe Asn Asp Lys Gln Gln Ile Ile Tyr Gly Lys Ile Pro Gly Met Val
385                 390                 395                 400

Val Asp Arg Cys Gly Cys Ser
                405

<210>  3
<211>  405
<212>  PRT
<213>  Artificial

<220>
<223>  Mus musculus

<400>  3

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Gly Val Gly Gly Glu Arg Ser Ser Arg
        35                  40                  45

Pro Ala Pro Ser Ala Pro Pro Glu Pro Asp Gly Cys Pro Val Cys Val
    50                  55                  60

Trp Arg Gln His Ser Arg Glu Leu Arg Leu Glu Ser Ile Lys Ser Gln
65                  70                  75                  80

Ile Leu Ser Lys Leu Arg Leu Lys Glu Ala Pro Asn Ile Ser Arg Glu
                85                  90                  95

Val Val Lys Gln Leu Leu Pro Lys Ala Pro Pro Leu Gln Gln Ile Leu
            100                 105                 110

Asp Leu His Asp Phe Gln Gly Asp Ala Leu Gln Pro Glu Asp Phe Leu
        115                 120                 125

Glu Glu Asp Glu Tyr His Ala Thr Thr Glu Thr Val Ile Ser Met Ala
    130                 135                 140

Gln Glu Thr Asp Pro Ala Val Gln Thr Asp Gly Ser Pro Leu Cys Cys
145                 150                 155                 160

His Phe His Phe Ser Pro Lys Val Met Phe Thr Lys Val Leu Lys Ala
                165                 170                 175

Gln Leu Trp Val Tyr Leu Arg Pro Val Pro Arg Pro Ala Thr Val Tyr
            180                 185                 190

Leu Gln Ile Leu Arg Leu Lys Pro Leu Thr Gly Glu Gly Thr Ala Gly
        195                 200                 205

Gly Gly Gly Gly Gly Arg Arg His Ile Arg Ile Arg Ser Leu Lys Ile
    210                 215                 220

Glu Leu His Ser Arg Ser Gly His Trp Gln Ser Ile Asp Phe Lys Gln
225                 230                 235                 240

Val Leu His Ser Trp Phe Arg Gln Pro Gln Ser Asn Trp Gly Ile Glu
                245                 250                 255

Ile Asn Ala Phe Asp Pro Ser Gly Thr Asp Leu Ala Val Thr Ser Leu
            260                 265                 270

Gly Pro Gly Ala Glu Gly Leu His Pro Phe Met Glu Leu Arg Val Leu
        275                 280                 285

Glu Asn Thr Lys Arg Ser Arg Arg Asn Leu Gly Leu Asp Cys Asp Glu

290                 295                 300

His Ser Ser Glu Ser Arg Cys Cys Arg Tyr Pro Leu Thr Val Asp Phe
305                 310                 315                 320

Glu Ala Phe Gly Trp Asp Trp Ile Ile Ala Pro Lys Arg Tyr Lys Ala
                325                 330                 335

Asn Tyr Cys Ser Gly Gln Cys Glu Tyr Met Phe Met Gln Lys Tyr Pro
            340                 345                 350

His Thr His Leu Val Gln Gln Ala Asn Pro Arg Gly Ser Ala Gly Pro
        355                 360                 365

Cys Cys Thr Pro Thr Lys Met Ser Pro Ile Asn Met Leu Tyr Phe Asn
    370                 375                 380

Asp Lys Gln Gln Ile Ile Tyr Gly Lys Ile Pro Gly Met Val Val Asp
385                 390                 395                 400

Arg Cys Gly Cys Ser
                405

<210>  4
<211>  405
<212>  PRT
<213>  Artificial

<220>
<223>  Pan troglodytes

<400>  4

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Gly Val Gly Gly Glu Arg Ser Ser Arg
        35                  40                  45

Pro Ala Pro Ser Val Ala Pro Glu Pro Asp Gly Cys Pro Val Cys Val
    50                  55                  60

Trp Arg Gln His Ser Arg Glu Leu Arg Leu Glu Ser Ile Lys Ser Gln
65                  70                  75                  80

Ile Leu Ser Lys Leu Arg Leu Lys Glu Ala Pro Asn Ile Ser Arg Glu
                85                  90                  95

Val Val Lys Gln Leu Leu Pro Lys Ala Pro Pro Leu Gln Gln Ile Leu
            100                 105                 110

Asp Leu His Asp Phe Gln Gly Asp Ala Leu Gln Pro Glu Asp Phe Leu
        115                 120                 125

Glu Glu Asp Glu Tyr His Ala Thr Thr Glu Thr Val Ile Ser Met Ala
    130                 135                 140

Gln Glu Thr Asp Pro Ala Val Gln Thr Asp Gly Ser Pro Leu Cys Cys
145                 150                 155                 160

His Phe His Phe Ser Pro Lys Val Met Phe Thr Lys Val Leu Lys Ala
                165                 170                 175

Gln Leu Trp Val Tyr Leu Arg Pro Val Pro Arg Pro Ala Thr Val Tyr
            180                 185                 190

Leu Gln Ile Leu Arg Leu Lys Pro Leu Thr Gly Glu Gly Thr Ala Gly
        195                 200                 205

Gly Gly Gly Gly Gly Arg Arg His Ile Arg Ile Arg Ser Leu Lys Ile
    210                 215                 220

Glu Leu His Ser Arg Ser Gly His Trp Gln Ser Ile Asp Phe Lys Gln
225                 230                 235                 240

Val Leu His Ser Trp Phe Arg Gln Pro Gln Ser Asn Trp Gly Ile Glu
                245                 250                 255

Ile Asn Ala Phe Asp Pro Ser Gly Thr Asp Leu Ala Val Thr Ser Leu
            260                 265                 270

Gly Pro Gly Ala Glu Gly Leu His Pro Phe Met Glu Leu Arg Val Leu
        275                 280                 285

Glu Asn Thr Lys Arg Ser Arg Arg Asn Leu Gly Leu Asp Cys Asp Glu
    290                 295                 300

His Ser Ser Glu Ser Arg Cys Cys Arg Tyr Pro Leu Thr Val Asp Phe
305                 310                 315                 320

Glu Ala Phe Gly Trp Asp Trp Ile Ile Ala Pro Lys Arg Tyr Lys Ala
                325                 330                 335

Asn Tyr Cys Ser Gly Gln Cys Glu Tyr Met Phe Met Gln Lys Tyr Pro

340                 345                 350

His Thr His Leu Val Gln Gln Ala Asn Pro Arg Gly Ser Ala Gly Pro
        355                 360                 365

Cys Cys Thr Pro Thr Lys Met Ser Pro Ile Asn Met Leu Tyr Phe Asn
    370                 375                 380

Asp Lys Gln Gln Ile Ile Tyr Gly Lys Ile Pro Gly Met Val Val Asp
385                 390                 395                 400

Arg Cys Gly Cys Ser
                405

<210>  5
<211>  407
<212>  PRT
<213>  Artificial

<220>
<223>  Gorilla gorilla

<400>  5

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Ala Gly Val Gly Gly Glu Arg Ser
        35                  40                  45

Ser Arg Pro Ala Pro Ser Val Ala Pro Glu Pro Asp Gly Cys Pro Val
    50                  55                  60

Cys Val Trp Arg Gln His Ser Arg Glu Leu Arg Leu Glu Ser Ile Lys
65                  70                  75                  80

Ser Gln Ile Leu Ser Lys Leu Arg Leu Lys Glu Ala Pro Asn Ile Ser
                85                  90                  95

Arg Glu Val Val Lys Gln Leu Leu Pro Lys Ala Pro Pro Leu Gln Gln
            100                 105                 110

Ile Leu Asp Leu His Asp Phe Gln Gly Asp Ala Leu Gln Pro Glu Asp
        115                 120                 125

Phe Leu Glu Glu Asp Glu Tyr His Ala Thr Thr Glu Thr Val Ile Ser
    130                 135                 140

Met Ala Gln Glu Thr Asp Pro Ala Val Gln Thr Asp Gly Ser Pro Leu
145                 150                 155                 160

Cys Cys His Phe His Phe Ser Pro Lys Val Met Phe Thr Lys Val Leu
                165                 170                 175

Lys Ala Gln Leu Trp Val Tyr Leu Arg Pro Val Pro Arg Pro Ala Thr
            180                 185                 190

Val Tyr Leu Gln Ile Leu Arg Leu Lys Pro Leu Thr Gly Glu Gly Thr
        195                 200                 205

Ala Gly Gly Gly Gly Gly Gly Arg Arg His Ile Arg Ile Arg Ser Leu
    210                 215                 220

Lys Ile Glu Leu His Ser Arg Ser Gly His Trp Gln Ser Ile Asp Phe
225                 230                 235                 240

Lys Gln Val Leu His Ser Trp Phe Arg Gln Pro Gln Ser Asn Trp Gly
                245                 250                 255

Ile Glu Ile Asn Ala Phe Asp Pro Ser Gly Thr Asp Leu Ala Val Thr
            260                 265                 270

Ser Leu Gly Pro Gly Ala Glu Gly Leu His Pro Phe Met Glu Leu Arg
        275                 280                 285

Val Leu Glu Asn Thr Lys Arg Ser Arg Arg Asn Leu Gly Leu Asp Cys
    290                 295                 300

Asp Glu His Ser Ser Glu Ser Arg Cys Cys Arg Tyr Pro Leu Thr Val
305                 310                 315                 320

Asp Phe Glu Ala Phe Gly Trp Asp Trp Ile Ile Ala Pro Lys Arg Tyr
                325                 330                 335

Lys Ala Asn Tyr Cys Ser Gly Gln Cys Glu Tyr Met Phe Met Gln Lys
            340                 345                 350

Tyr Pro His Thr His Leu Val Gln Gln Ala Asn Pro Arg Gly Ser Ala
        355                 360                 365

Gly Pro Cys Cys Thr Pro Thr Lys Met Ser Pro Ile Asn Met Leu Tyr
    370                 375                 380

Phe Asn Asp Lys Gln Gln Ile Ile Tyr Gly Lys Ile Pro Gly Met Val

385                 390                 395                 400

Val Asp Arg Cys Gly Cys Ser
                405

<210>  6
<211>  406
<212>  PRT
<213>  Artificial

<220>
<223>  Rhinopithecus roxellana

<400>  6

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Gly Val Gly Gly Glu Arg Ser Ser
        35                  40                  45

Arg Pro Ala Pro Ser Val Ala Pro Glu Pro Asp Gly Cys Pro Val Cys
    50                  55                  60

Val Trp Arg Gln His Ser Arg Glu Leu Arg Leu Glu Ser Ile Lys Ser
65                  70                  75                  80

Gln Ile Leu Ser Lys Leu Arg Leu Lys Glu Ala Pro Asn Ile Ser Arg
                85                  90                  95

Glu Val Val Lys Gln Leu Leu Pro Lys Ala Pro Pro Leu Gln Gln Ile
            100                 105                 110

Leu Asp Leu His Asp Phe Gln Gly Asp Ala Leu Gln Pro Glu Asp Phe
        115                 120                 125

Leu Glu Glu Asp Glu Tyr His Ala Thr Thr Glu Thr Val Ile Ser Met
    130                 135                 140

Ala Gln Glu Thr Asp Pro Ala Val Gln Thr Asp Gly Ser Pro Leu Cys
145                 150                 155                 160

Cys His Phe His Phe Ser Pro Lys Val Met Phe Thr Lys Val Leu Lys
                165                 170                 175

Ala Gln Leu Trp Val Tyr Leu Arg Pro Val Pro Arg Pro Ala Thr Val
            180                 185                 190

Tyr Leu Gln Ile Leu Arg Leu Lys Pro Leu Thr Gly Glu Gly Thr Ala
        195                 200                 205

Gly Gly Gly Gly Gly Gly Arg Arg His Ile Arg Ile Arg Ser Leu Lys
    210                 215                 220

Ile Glu Leu His Ser Arg Ser Gly His Trp Gln Ser Ile Asp Phe Lys
225                 230                 235                 240

Gln Val Leu His Ser Trp Phe Arg Gln Pro Gln Ser Asn Trp Gly Ile
                245                 250                 255

Glu Ile Asn Ala Phe Asp Pro Ser Gly Thr Asp Leu Ala Val Thr Ser
            260                 265                 270

Leu Gly Pro Gly Ala Glu Gly Leu His Pro Phe Met Glu Leu Arg Val
        275                 280                 285

Leu Glu Asn Thr Lys Arg Ser Arg Arg Asn Leu Gly Leu Asp Cys Asp
    290                 295                 300

Glu His Ser Ser Glu Ser Arg Cys Cys Arg Tyr Pro Leu Thr Val Asp
305                 310                 315                 320

Phe Glu Ala Phe Gly Trp Asp Trp Ile Ile Ala Pro Lys Arg Tyr Lys
                325                 330                 335

Ala Asn Tyr Cys Ser Gly Gln Cys Glu Tyr Met Phe Met Gln Lys Tyr
            340                 345                 350

Pro His Thr His Leu Val Gln Gln Ala Asn Pro Arg Gly Ser Ala Gly
        355                 360                 365

Pro Cys Cys Thr Pro Thr Lys Met Ser Pro Ile Asn Met Leu Tyr Phe
    370                 375                 380

Asn Asp Lys Gln Gln Ile Ile Tyr Gly Lys Ile Pro Gly Met Val Val
385                 390                 395                 400

Asp Arg Cys Gly Cys Ser
                405

<210>  7
<211>  409
<212>  PRT
<213>  Artificial

<220>
<223>  Nomascus leucogenys

<400>  7

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Ala Ala Ala Gly Val Gly Gly Glu
        35                  40                  45

Arg Ser Ser Arg Pro Ala Pro Ser Val Ala Pro Glu Pro Asp Gly Cys
    50                  55                  60

Pro Val Cys Val Trp Arg Gln His Ser Arg Glu Leu Arg Leu Glu Ser
65                  70                  75                  80

Ile Lys Ser Gln Ile Leu Ser Lys Leu Arg Leu Lys Glu Ala Pro Asn
                85                  90                  95

Ile Ser Arg Glu Val Val Lys Gln Leu Leu Pro Lys Ala Pro Pro Leu
            100                 105                 110

Gln Gln Ile Leu Asp Leu His Asp Phe Gln Gly Asp Ala Leu Gln Pro
        115                 120                 125

Glu Asp Phe Leu Glu Glu Asp Glu Tyr His Ala Thr Thr Glu Thr Val
    130                 135                 140

Ile Ser Met Ala Gln Glu Thr Asp Pro Ala Val Gln Thr Asp Gly Ser
145                 150                 155                 160

Pro Leu Cys Cys His Phe His Phe Ser Pro Lys Val Met Phe Thr Lys
                165                 170                 175

Val Leu Lys Ala Gln Leu Trp Val Tyr Leu Arg Pro Val Pro Arg Pro
            180                 185                 190

Ala Thr Val Tyr Leu Gln Ile Leu Arg Leu Lys Pro Leu Thr Gly Glu
        195                 200                 205

Gly Thr Ala Gly Ala Gly Gly Gly Gly Arg Arg His Ile Arg Ile Arg
    210                 215                 220

Ser Leu Lys Ile Glu Leu His Ser Arg Ser Gly His Trp Gln Ser Ile
225                 230                 235                 240

Asp Phe Lys Gln Val Leu His Ser Trp Phe Arg Gln Pro Gln Ser Asn
                245                 250                 255

Trp Gly Ile Glu Ile Asn Ala Phe Asp Pro Ser Gly Thr Asp Leu Ala
            260                 265                 270

Val Thr Ser Leu Gly Pro Gly Ala Glu Gly Leu His Pro Phe Met Glu
        275                 280                 285

Leu Arg Val Leu Glu Asn Thr Lys Arg Ser Arg Arg Asn Leu Gly Leu
    290                 295                 300

Asp Cys Asp Glu His Ser Ser Glu Ser Arg Cys Cys Arg Tyr Pro Leu
305                 310                 315                 320

Thr Val Asp Phe Glu Ala Phe Gly Trp Asp Trp Ile Ile Ala Pro Lys
                325                 330                 335

Arg Tyr Lys Ala Asn Tyr Cys Ser Gly Gln Cys Glu Tyr Met Phe Met
            340                 345                 350

Gln Lys Tyr Pro His Thr His Leu Val Gln Gln Ala Asn Pro Arg Gly
        355                 360                 365

Ser Ala Gly Pro Cys Cys Thr Pro Thr Lys Met Ser Pro Ile Asn Met
    370                 375                 380

Leu Tyr Phe Asn Asp Lys Gln Gln Ile Ile Tyr Gly Lys Ile Pro Gly
385                 390                 395                 400

Met Val Val Asp Arg Cys Gly Cys Ser
                405

<210> 8
<211> 408
<212> PRT
<213> Artificial

<220>
<223> Macaca mulatta

<400> 8

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Ala Ala Ala Ala Gly Val Gly Gly Glu Arg
        35                  40                  45

Ser Ser Arg Pro Ala Pro Ser Val Ala Pro Glu Pro Asp Gly Cys Pro
    50                  55                  60

Val Cys Val Trp Arg Gln His Ser Arg Glu Leu Arg Leu Glu Ser Ile
65                  70                  75                  80

Lys Ser Gln Ile Leu Ser Lys Leu Arg Leu Lys Glu Ala Pro Asn Ile
                85                  90                  95

Ser Arg Glu Val Val Lys Gln Leu Leu Pro Lys Ala Pro Pro Leu Gln
            100                 105                 110

Gln Ile Leu Asp Leu His Asp Phe Gln Gly Asp Ala Leu Gln Pro Glu
        115                 120                 125

Asp Phe Leu Glu Glu Asp Glu Tyr His Ala Thr Thr Glu Thr Val Ile
    130                 135                 140

Ser Met Ala Gln Glu Thr Asp Pro Ala Val Gln Thr Asp Gly Ser Pro
145                 150                 155                 160

Leu Cys Cys His Phe His Phe Ser Pro Lys Val Met Phe Thr Lys Val
                165                 170                 175

Leu Lys Ala Gln Leu Trp Val Tyr Leu Arg Pro Val Pro Arg Pro Ala
            180                 185                 190

Thr Val Tyr Leu Gln Ile Leu Arg Leu Lys Pro Leu Thr Gly Glu Gly
        195                 200                 205

Thr Ala Gly Gly Gly Gly Gly Gly Arg Arg His Ile Arg Ile Arg Ser
    210                 215                 220

Leu Lys Ile Glu Leu His Ser Arg Ser Gly His Trp Gln Ser Ile Asp
225                 230                 235                 240

Phe Lys Gln Val Leu His Ser Trp Phe Arg Gln Pro Gln Ser Asn Trp
                245                 250                 255

Gly Ile Glu Ile Asn Ala Phe Asp Pro Ser Gly Thr Asp Leu Ala Val
            260                 265                 270

Thr Ser Leu Gly Pro Gly Ala Glu Gly Leu His Pro Phe Met Glu Leu
        275                 280                 285

Arg Val Leu Glu Asn Thr Lys Arg Ser Arg Arg Asn Leu Gly Leu Asp
    290                 295                 300

Cys Asp Glu His Ser Ser Glu Ser Arg Cys Cys Arg Tyr Pro Leu Thr
305                 310                 315                 320

Val Asp Phe Glu Ala Phe Gly Trp Asp Trp Ile Ile Ala Pro Lys Arg
                325                 330                 335

Tyr Lys Ala Asn Tyr Cys Ser Gly Gln Cys Glu Tyr Met Phe Met Gln
            340                 345                 350

Lys Tyr Pro His Thr His Leu Val Gln Gln Ala Asn Pro Arg Gly Ser
        355                 360                 365

Ala Gly Pro Cys Cys Thr Pro Thr Lys Met Ser Pro Ile Asn Met Leu
    370                 375                 380

Tyr Phe Asn Asp Lys Gln Gln Ile Ile Tyr Gly Lys Ile Pro Gly Met
385                 390                 395                 400

Val Val Asp Arg Cys Gly Cys Ser
                405

<210>  9
<211>  263
<212>  PRT
<213>  Artificial

<220>
<223>  Pan paniscus

<400>  9

Met Ala Gln Glu Thr Asp Pro Ala Val Gln Thr Asp Gly Ser Pro Leu
1               5                   10                  15

Cys Cys His Phe His Phe Ser Pro Lys Val Met Phe Thr Lys Val Leu
            20                  25                  30

Lys Ala Gln Leu Trp Val Tyr Leu Arg Pro Val Pro Arg Pro Ala Thr
        35                  40                  45

Val Tyr Leu Gln Ile Leu Arg Leu Lys Pro Leu Thr Gly Glu Gly Thr
    50                  55                  60

Ala Gly Gly Gly Gly Gly Gly Arg Arg His Ile Arg Ile Arg Ser Leu
65                  70                  75                  80

Lys Ile Glu Leu His Ser Arg Ser Gly His Trp Gln Ser Ile Asp Phe
                85                  90                  95

Lys Gln Val Leu His Ser Trp Phe Arg Gln Pro Gln Ser Asn Trp Gly
            100                 105                 110

Ile Glu Ile Asn Ala Phe Asp Pro Ser Gly Thr Asp Leu Ala Val Thr
        115                 120                 125

Ser Leu Gly Pro Gly Ala Glu Gly Leu His Pro Phe Met Glu Leu Arg
    130                 135                 140

Val Leu Glu Asn Thr Lys Arg Ser Arg Arg Asn Leu Gly Leu Asp Cys
145                 150                 155                 160

Asp Glu His Ser Ser Glu Ser Arg Cys Cys Arg Tyr Pro Leu Thr Val
                165                 170                 175

Asp Phe Glu Ala Phe Gly Trp Asp Trp Ile Ile Ala Pro Lys Arg Tyr
            180                 185                 190

Lys Ala Asn Tyr Cys Ser Gly Gln Cys Glu Tyr Met Phe Met Gln Lys
        195                 200                 205

Tyr Pro His Thr His Leu Val Gln Gln Ala Asn Pro Arg Gly Ser Ala
    210                 215                 220

Gly Pro Cys Cys Thr Pro Thr Lys Met Ser Pro Ile Asn Met Leu Tyr
225                 230                 235                 240

Phe Asn Asp Lys Gln Gln Ile Ile Tyr Gly Lys Ile Pro Gly Met Val
                245                 250                 255

Val Asp Arg Cys Gly Cys Ser
            260

<210>  10
<211>  404
<212>  PRT
<213>  Artificial

<220>
<223>  Sus scrofa domesticus

<400>  10

Met Val Leu Ala Ala Pro Leu Leu Leu Gly Phe Leu Leu Leu Ala Leu
1               5                   10                  15

Glu Leu Arg Pro Arg Gly Glu Ala Ala Glu Gly Pro Ala Ala Ala Ala
            20                  25                  30

Ala Ala Ala Ala Ala Ala Gly Ala Gly Gly Glu Arg Ser Ser Arg Pro
        35                  40                  45

Ala Ala Ser Val Ala Pro Glu Pro Asp Gly Cys Pro Val Cys Leu Trp
    50                  55                  60

Arg Gln His Ser Arg Glu Leu Arg Leu Glu Ser Val Lys Ser Gln Ile
65                  70                  75                  80

Leu Ser Lys Leu Arg Leu Lys Glu Ala Pro Asn Ile Ser Arg Glu Val
                85                  90                  95

Val Lys Gln Leu Leu Pro Lys Ala Pro Pro Leu Gln Gln Ile Leu Asp
            100                 105                 110

Leu His Asp Phe Gln Gly Asp Ala Leu Gln Pro Glu Asp Phe Leu Glu
        115                 120                 125

Glu Asp Glu Tyr His Ala Thr Thr Glu Thr Val Ile Ser Met Ala Gln
    130                 135                 140

Glu Thr Asp Pro Ala Val Gln Thr Asp Gly Ser Pro Leu Cys Cys His
145                 150                 155                 160

Phe His Phe Ser Pro Lys Val Met Phe Thr Lys Val Leu Lys Ala Gln
                165                 170                 175

Leu Trp Val Tyr Leu Arg Pro Val Pro Arg Pro Ala Thr Val Tyr Leu
            180                 185                 190

Gln Ile Leu Arg Leu Lys Pro Leu Thr Gly Glu Gly Thr Ala Gly Gly
        195                 200                 205

Gly Gly Gly Gly Arg Arg His Ile Arg Ile Arg Ser Leu Lys Ile Asp
    210                 215                 220

Leu His Ser Arg Ser Gly His Trp Gln Ser Ile Asp Phe Lys Gln Val
225                 230                 235                 240

Leu His Ser Trp Phe Arg Gln Pro Gln Ser Asn Trp Gly Ile Glu Ile
                245                 250                 255

Asn Ala Phe Asp Pro Ser Gly Thr Asp Leu Ala Val Thr Ser Leu Gly
            260                 265                 270

Pro Gly Ala Glu Gly Leu His Pro Phe Met Glu Leu Arg Val Leu Glu
        275                 280                 285

Asn Thr Lys Arg Ser Arg Arg Asn Leu Gly Leu Asp Cys Asp Glu His
    290                 295                 300

Ser Ser Glu Ser Arg Cys Cys Arg Tyr Pro Leu Thr Val Asp Phe Glu
305                 310                 315                 320

Ala Phe Gly Trp Asp Trp Ile Ile Ala Pro Lys Arg Tyr Lys Ala Asn
                325                 330                 335

Tyr Cys Ser Gly Gln Cys Glu Tyr Met Phe Met Gln Lys Tyr Pro His
            340                 345                 350

Thr His Leu Val Gln Gln Ala Asn Pro Arg Gly Ser Ala Gly Pro Cys
        355                 360                 365

Cys Thr Pro Thr Lys Met Ser Pro Ile Asn Met Leu Tyr Phe Asn Asp
    370                 375                 380

Lys Gln Gln Ile Ile Tyr Gly Lys Ile Pro Gly Met Val Val Asp Arg
385                 390                 395                 400

Cys Gly Cys Ser

<210>  11
<211>  330
<212>  DNA
<213>  Artificial

<220>
<223>  GDF-11 polynucleotide

<400>  11
aacctgggtc tggactgcga cgagcactca agcgagtccc gctgctgccg atatcccctc      60

acagtggact ttgaggcttt cggctgggac tggatcatcg cacctaagcg ctacaaggcc     120

aactactgct ccggccagtg cgagtacatg ttcatgcaaa aatatccgca tacccatttg     180

gtgcagcagg ccaatccaag aggctctgct gggccctgtt gtacccccac caagatgtcc     240

ccaatcaaca tgctctactt caatgacaag cagcagatta tctacggcaa gatccctggc     300

atggtggtgg atcgctgtgg ctgctcttaa                                      330

## Description

### [Technical Field]

The present invention relates to a novel therapeutic pharmaceutical composition, and more specifically, the present invention relates to a pharmaceutical composition for treating TRPV1-mediated diseases and a method for treating TRPV1-mediated diseases.

### [Background Art]

Pain is 'an unpleasant sensory and emotional experience associated with actual or potential tissue damage', and in general, pain is relieved as the damaged tissue recovers, but when pain becomes chronic, pain continues to occur without any irritation, even though the damaged area has clearly healed completely, and pain may be caused by harmless stimuli that do not generally cause pain.

Meanwhile, transient receptor potential vanilloid type 1 (TRPV1) is a member of the transient receptor potential (TRP) family as one of the important nociceptive receptors in the cellular mechanism by which noxious stimulation causes pain. In addition, TRPV1 is a non-selective cation channel that is activated by various stimuli such as noxious heat (42°C or more), capsaicin, resiniferatoxin (RTX), protons, and the like, and it is a key molecule that is mainly expressed in the terminal of a nociceptive nerve fibers and is involved in the development of peripheral and central sensitization that causes chronic pain. TRPV1 has been suggested as a major target for the treatment of chronic pain, but pharmacological inhibition of TRPV1 causes severe high fever with unknown cause, and it is known that the development of a TRPV1 inhibitor as a therapeutic agent is difficult.

GDF11 is a protein belonging to the TGF-β family and is known to increase expression in young animals to promote neurogenesis and angiogenesis. Many studies have been conducted on the control effect of chronic pain of TGF-β belonging to the same family, but the part regarding the relief effect of chronic pain of GDF11 remains unknown.

Korean Patent Publication No. 10-2017-0093286 relates to a composition including GDF11 and a use thereof, and provides a fibroblast proliferation effect of a human-derived adult stem cell culture solution including GDF11. However, there are no studies or disclosures related to the chronic pain relief effect of GDF11.

### [Disclosure]

### [Technical Problem]

As a result of making diligent efforts to provide a substance that can resolve side effects such as severe high fever and the like that accompany TRPV1 inhibition while treating chronic pain through TRPV1 inhibition, the present inventors completed the present invention by confirming that GDF11 has an effect of relieving pain that occurs by various causes.

Accordingly, an object of the present invention is to provide a pharmaceutical composition for treating TRPV1-mediated diseases and pain-related diseases including chronic pain without side effects. However, these problems are exemplary, and the scope of the present invention is not limited thereto.

### [Technical Solution]

According to one aspect of the present invention, provided is a pharmaceutical composition for treating TRPV1-mediated disease, comprising a growth differentiation factor 11 (GDF11) peptide comprising an amino acid sequence of SEQ ID NO: 1, a polynucleotide encoding the peptide or an expression vector comprising the polynucleotide as an active ingredient.

According to another aspect of the present invention, provided is a method for treating TRPV1-mediated disease, comprising administering a composition comprising growth differentiation factor 11 (GDF11) peptide comprising an amino acid sequence of SEQ ID NO: 1, a polynucleotide encoding the peptide or an expression vector comprising the polynucleotide to a patient with TRPV1-mediated disease.

### Definition of terms:

As used herein, the term "growth differentiation factor 11 (GDF11)", also referred to as bone morphogenetic protein 11 (BMP11), refers to a protein expressed by the GDF11 gene present on chromosome 12 in humans.

As used herein, the term "transient receptor potential channel vanilloid subfamily member 1 (TRPV1)" belongs to a large family of TRP channels consisting of non-voltage gated cation channels including heat, visual, taste, olfactory, and tactile areas. TRPV1 is activated synergistically by interacting heat, protons, and endogenous substances to trigger nociceptive signals.

As used herein, the term "vector" refers to a tool used for gene delivery. It can be deemed as a concept corresponding to the carrier of a drug. Therefore, vectors in a broad sense include not only DNA vectors composed of the same nucleic acid molecules as transgenes to be introduced similar to general plasmid vectors, but also nanoparticles made of silica or gold, liposomes, extracellular vesicles, and phospholipid membrane structures such as exosomes, chitosan, cationic polymers such as polyethylene imine and polylysine, and reagents used for DNA transfection such as calcium phosphate.

### Detailed description of the invention:

According to one aspect of the present invention, provided is a pharmaceutical composition for treating TRPV1-mediated disease, comprising: a growth differentiation factor 11 (GDF11) peptide comprising an amino acid sequence of SEQ ID NO: 1, a polynucleotide encoding the peptide or an expression vector comprising the polynucleotide as an active ingredient.

In the pharmaceutical composition, the expression vector may be a viral vector or a non-viral vector, and the viral vector may be an adeno-associated virus (AAV) vector, an adenovirus vector, an alphavirus vector, a herpes simplex virus vector, a vaccinia vector, a Sendai virus vector, a flavivirus vector, a rhabdovirus vector, a retrovirus vector, or a lentivirus vector. Also, the serotype of the adeno-associated virus (AAV) vector may be AAV1, AAV2, AAV3, AAV4, AAV5, AAV6, AAV7, AAV8, AAV9, AAV10, AAV11, AAV12, AAV13, AAV14, AAV15, or AAV16.

In the pharmaceutical composition, the non-viral vector may be a DNA vector, a nanoparticle, a cationic polymer, an exosome, an extracellular vesicle, or a liposome, and the DNA vector may be a plasmid vector, a cosmid vector, a phagemid vector, or an artificial human chromosome.

In the pharmaceutical composition, the TRPV1-mediated disease may be selected from the group consisting of pain, hypertension, stroke, myocardial ischemia, urinary incontinence, urinary bladder hypersensitiveness, irritable bowel syndrome, fecal urgency, stomach-duodenal ulcer, gastro-esophageal reflux disease (GERD), Crohn's disease, haemorrhoid, asthma, chronic obstructive pulmonary disease, pruritus, psoriasis, hearing loss, tinnitus, cough, hypertrichosis, and alopecia.

In the pharmaceutical composition, the pain may be nociceptive pain, psychogenic pain, inflammatory pain, or pathological pain, and the pathological pain may be neuropathic pain, cancer pain, chemotherapy-induced pain, postoperative pain, trigeminal neuralgia pain, idiopathic pain, diabetic neuropathic pain, or migraine.

According to another aspect of the present invention, provided is a method for alleviating or treating pain, comprising administering the pharmaceutical composition.

Pain according to the present invention includes nociceptive pain, psychogenic pain, inflammatory pain associated with tissue damage and invasion of immune cells, pathological pain (dysfunctional pain such as fibromyalgia, irritable bowel syndrome, and tension headache), which is a disease state caused by damage to the nervous system or its abnormal function. In addition, pain may include back pain that is anatomically distinct. Pain may include pain such as neuropathic pain, migraine, and the like, as well as neck pain, middle back pain, lower back pain, or tailbone pain. Neuropathic pain is a chronic neurological disease that is caused when the nervous system is damaged by various causes such as trauma, inflammation, ischemic damage, metabolites, or the like, and it may be caused by damage or disease that affects the somatosensory system. In general, it is a type of non-malignant chronic pain that is caused by abnormalities in the nerves, spinal cord, and brain, and it is estimated that more than 1% of the population suffers therefrom.

Neuropathic pain may be associated with an abnormal sensation called dysesthesia, allodynia in which pain is felt even with harmless stimuli that do not cause pain, and hyperalgesia in which pain is felt more intensely and longer in response to harmful stimuli such as fever. In addition, neuropathic pain may be a continuous and/or intermittent (seizure) factor. The latter is likened to an electric shock. Common traits include burning, cold, pins and needles, numbness, and itch. Depending on whether the peripheral or central nervous system is affected, it may be classified into peripheral neuropathic pain and central neuropathic pain. In contrast, nociceptive pain is often expressed as ache. In addition, migraine is a chronic disorder that is associated with a number of symptoms of the autonomic nervous system and causes headaches ranging from normal to severe. The exact mechanisms of these migraine headaches have not been identified to date. The basic theory is associated with increased excitability in the cerebral cortex and abnormal regulation of pain neurons in the trigeminal nucleus of the brain stem. For example, pain may be one or more selected from the group consisting of neuropathic pain, cancer pain, postoperative pain, trigeminal neuralgia pain, idiopathic pain, diabetic neuropathic pain, migraine, and the like.

In the pharmaceutical composition of the present invention, the effective amount of the compound may vary depending on the type of the affected part of the patient, the application site, the number of treatments, the treatment time, the formulation, the condition of the patient, the type of adjuvants, and the like. The amount used is not particularly limited, but may be 0.01 µg/kg/day to 10 mg/kg/day. The daily dose may be administered once a day, administered by dividing into 2 to 3 times a day at appropriate intervals, or intermittently administered at intervals of several days.

In the pharmaceutical composition of the present invention, the compound may be contained at an amount of 0.01 wt.% to 100 wt.% based on the total weight of the composition. The pharmaceutical composition of the present invention may further include suitable carriers, excipients, and diluents commonly used in the preparation of pharmaceutical compositions. In addition, solid or liquid additives for formulation may be used in the preparation of pharmaceutical compositions. The additive for formulation may be organic or inorganic. Examples of excipients include lactose, sucrose, white sugar, glucose, corn starch, starch, talc, sorbitol, crystalline cellulose, dextrin, kaolin, calcium carbonate, silicon dioxide, and the like. Examples of binders include polyvinyl alcohol, polyvinyl ether, ethyl cellulose, methyl cellulose, gum Arabic, tragacanth, gelatin, shellac, hydroxypropyl cellulose, hydroxypropyl methyl cellulose, calcium citrate, dextrin, pectin, and the like. Examples of lubricants include magnesium stearate, talc, polyethylene glycol, silica, hydrogenated vegetable oil, and the like. Any colorant that is permitted to be added to conventional pharmaceuticals may be used. These tablets and granules may be appropriately coated with a sugar coat and gelatin coating depending on other needs. In addition, preservatives, antioxidants, and the like may be added as necessary.

The pharmaceutical composition of the present invention may be prepared in any formulation conventionally prepared in the art, and the form of the formulation is not particularly limited.

The pharmaceutical composition of the present invention may be administered orally or parenterally, and preferably, parenteral administration may be intravenous injection, subcutaneous injection, intracerebroventricular injection, intra-cerebrospinal fluid injection, intrathecal injection, transforaminal injection, intramuscular injection, intraperitoneal injection, and the like.

The present invention provides a method of alleviating and/or treating pain comprising administering a therapeutically effective amount of the pharmaceutical composition to a patient in need of alleviating and/or treating pain. The method may further include a step of identifying a patient as the patient in need of pain relief and/or treatment prior to the administering step. The term "therapeutically effective amount" may depend on the amount of an active gradient that can achieve the desired effect, pain relief, and/or therapeutic effect.

The pharmaceutical composition of the present invention is useful for the prevention and treatment of diseases related to the activity of TRPV1, including pain such as acute pain, chronic pain, neuropathic pain, postoperative pain, rheumatoid arthritis pain, arthritis pain, posterior herpes neuralgia, neuralgia, headache, toothache, pelvic pain, migraine, bone cancer pain, chemotherapy-induced pain, breast pain and visceral pain; nerve-related diseases such as neuropathy, HIV-related neuropathy, nerve injury, neurodegeneration, and stroke; diabetic peripheral neuropathy; fecal urgency; irritable bowel syndrome; inflammatory bowel disease; gastrointestinal diseases such as gastroesophageal reflux disease (GERD), gastric duodenal ulcer, and Crohn's disease; respiratory diseases such as asthma, chronic obstructive pulmonary disease, and cough; urinary incontinence; urinary bladder hypersensitivity; neurotic/allergic/inflammatory skin diseases such as psoriasis, pruritus, prurigo, and dermatitis; hyperacusis; hearing loss; tinnitus; vestibular hypersensitiveness; heart diseases such as myocardial ischemia; hemorrhagic shock; hair growth-related diseases such as hirsutism, effluvium, and alopecia; rhinitis; pancreatitis; cystitis; vulvodynia; mental disorders such as tension or fear; obesity; type 1 diabetes and type 2 diabetes, but is not limited thereto.

GDF11 of the present invention is a protein encoded by the 12^{th} gene of the growth differentiation factor, and GDF11 serves as a cytokine and has the same molecular structure in humans and mice. The bone morphogenetic protein group is characterized by a polybasic proteolytic processing site and is cleaved to produce a protein containing 7 conserved cysteine residues. Systemic GDF11 treatment improves the vascular structure of the hippocampus and cortex in elderly mice to improve neurogenesis, and systematic supplementation of GDF11 improved the survival and morphology of β-cells in both nongenetic and genetic mouse models of type 2 diabetes, and improved glucose metabolism.

GDF11 induces phenotypes such as calorie restriction without affecting appetite or GDF15 levels in the blood, restores insulin/IGF-1 signaling pathways, acts directly on fat cells to stimulate the adiponectin secretion from white adipose tissues, and restores neurogenesis in the aged brain. In addition, it is a regulator of skin biology, has an important effect on the production of procollagen I and hyaluronic acid, activates the Smad2/3 phosphorylation pathway in skin endothelial cells, and improves skin vasculature. Increased GDF11 levels in aged mice also improved muscle structures and functional characteristics and increased strength and endurance exercise capacity.

Moreover, GDF11 was found to reduce oxidative stress, reduce levels of AGE, protein oxidation, and lipid peroxidation, and slow the accumulation of age-related histologic markers, and it significantly inhibited the reduction of CAT, GPX, and SOD activities. A member of the GDF11 family is a regulation factor of cell growth and differentiation in both embryonic and adult tissues, and studies on mice and African clawed frogs (the genus Xenopus) reported that the protein is involved in mesoderm formation and neurogenesis during embryonic development.

Human GDF11 represented by SEQ ID NO: 2 and GDF11 derived from other mammals having at least 96% or more homology thereto may also be used. The other mammal-derived GDF11 may be a mouse (Mus musculus, SEQ ID NO: 3), a chimpanzee (Pan troglodytes, SEQ ID NO: 4), a gorilla (Gorilla beringei graueri, SEQ ID NO: 5), a golden snub-nosed monkey *(Rhinopithecus roxellana*, SEQ ID NO: 6), a Northern white-cheeked gibbon *(Nomascus leucogenys,* SEQ ID NO: 7), a Bengal monkey (Macaca mulatta, SEQ ID NO: 8), a bonobo (Pan paniscus, SEQ ID NO: 9), or a pig (Sus scrofa domesticus, SEQ ID NO: 10).

The human-derived GDF11 protein is a protein having very high homology with other primate-derived GDF11, and all or part of full-length peptides or mature peptides derived from other animals may be used. Among these, mature peptides correspond to amino acid positions 299 to 407 (SEQ ID NO: 1), and amino acid positions 1 to 298 are a portion that is cleaved among signal peptides and precursor proteins. Since the mature peptides have almost 100% homology with other mammals, genes or proteins derived from other animals may be used. In addition, the polynucleotide encoding the mature peptide may be represented by SEQ ID NO: 11.

The present invention may be implemented by gene therapy using genes instead of proteins or peptides. For such gene therapy, in order to facilitate gene amplification and manipulation, a recombinant vector in which a gene construct in which a polynucleotide encoding GDF11 is operably linked to a regulatory sequence is inserted into various expression vectors is used. Such an expression vector is classified into a viral vector and a non-viral vector. For the viral vector, an adeno-associated virus (AAV) vector, an adenovirus vector, an alphavirus vector, a herpes simplex virus vector, a vaccinia vector, a Sendai virus vector, a flavivirus vector, a rhabdovirus vector, a retrovirus vector, a lentivirus vector, and the like are being used. Viral vectors used in gene therapy are well documented in the literature. The above literature is incorporated herein by reference. However, in the case of a retrovirus or a lentivirus among these viral vectors, a transgene is inserted into the genome of a host, but since it is inserted randomly, unexpected side effects such as the occurrence of cancer are concerned. Accordingly, adeno-associated virus, which is inserted only at a certain location on the genome of a host cell, has received attention.

The adeno-associated virus (AAV) is a single-stranded DNA virus and is a Helper-dependent human parvovirus. The genome size is about 4.7 kbp, and the N-terminal portion of the genome encodes the rep gene involved in viral replication and expression of viral genes, and the C-terminal portion encodes the cap gene, which encodes the viral capsid protein. Also, it is composed of an inverted terminal repeat (ITR) in which approximately 145 bases are inserted at both ends. Four proteins are translated from the rep region, which are classified into rep78, rep68, rep52, and rep40 according to the molecular weight, and play an important function in AAV DNA replication. From the cap region, three proteins, that is, VP1, VP2, and VP3, are translated, and these are structural proteins required for the AAV virus assembly.

The AAV vector of the present invention may be used to transport foreign gene sequences into cells according to various viral infection methods known in the art, and the method is not particularly limited.

Meanwhile, the expression vector for gene therapy according to an exemplary embodiment of the present invention may be inserted into a DNA vector such as a non-viral expression vector including a gene construct in which a polynucleotide encoding GDF11 is operably linked to a regulatory sequence, in particular, a plasmid vector and the like.

As used herein, the term "operably linked to" means that a target nucleic acid sequence is linked to the regulatory sequence in a manner that allows the expression in the living body to which a transgene is to be introduced.

The term "regulatory sequence" means to include promoters, enhancers, and other regulatory elements (e.g., polyadenylation signals). The regulatory sequence includes instructing that the target nucleic acid can always be expressed in many host cells, instructing that the target nucleic acid can be expressed only in cells of a specific tissue (e.g., tissue-specific regulatory sequence), and instructing that the expression is induced by a specific signal *(e.g.,* an inducible regulatory sequence). It can be understood by those skilled in the art that the design of an expression vector used in gene therapy may vary depending on factors such as the selection of a host into which the transgene is to be introduced, the level of desired protein expression, and the like. The expression vector of the present invention may be introduced into a host to express the GDF11 protein. Therefore, the expression vector according to an exemplary embodiment of the present invention must have a regulatory sequence of a eukaryotic cell capable of expressing a foreign gene in eukaryotic cells, particularly, mammals. The regulatory sequences that enable the expression of foreign genes in such eukaryotic cells are well known to those in the corresponding industry. As described above, they usually include regulatory sequences responsible for initiation of transcription and optionally include a poly-A signal responsible for termination and stabilization of transcription of a transcript. Additional regulatory sequences may include translation enhancing factors and/or naturally-combined or heterologous promoter regions in addition to transcriptional regulators. For example, regulatory sequences that allow expression in cells in mammals are the CMV-HSV thymidine kinase promoter, SV40, an RSV-promoter (Rous sarcoma virus), a human kidney element 1α-promoter, a glucocorticoid-inducible MMTV-promoter (Moloni mouse tumor virus), a metallothionein-inducible or tetracycline-inducible promoter, or an amplifying agent such as a CMV amplifying agent or an SV40-amplifying agent. For expression in neurons, it is considered that a neurofilament-promoter, a PGDF-promoter, an NSE-promoter, a PrP-promoter, or a thy-1-promoter may be used. Such promoters are known in the art and are described in the literature. The regulatory sequences may include a transcription termination signal such as an SV40-poly-A site or a TK-poly-A site downstream of the polynucleotide according to an exemplary embodiment of the present invention. In the present invention, suitable expression vectors are known in the art, and examples thereof include Okayama-Berg cDNA expression vector pcDV1 (Parmacia), pRc/CMV, pcDNA1, pcDNA3 (Invitrogen), pSPORT1 (GIBCO BRL), pGX-27, pX, yeast two-hybrid vectors, and for example, pEG202, dpJG4-5, and the like. In addition to the nucleic acid molecules of the present invention, the vector may further include a polynucleotide encoding a secretion signal. These secretion signals are well known to those in the corresponding industry. In addition, depending on the expression system used, a leader sequence capable of leading GDF11 to the cell compartment may be combined with the coding sequence of the polynucleotide according to an exemplary embodiment of the present invention, and preferably, it is a leader sequence capable of directly secreting the translated protein or its protein into the periplasm or the extracellular matrix.

In addition, the expression vector of the present invention may be prepared by, for example, standard recombinant DNA techniques, and the standard recombinant DNA techniques include, for example, ligation of blunt ends and adhesive ends, treatment with restriction enzymes to provide appropriate ends, phosphate group removal by alkaline phosphatase treatment in order to prevent inappropriate binding, enzymatic linkage by T4 DNA ligase, and the like. The vector of the present invention may be prepared by recombining DNA encoding a signal peptide obtained by chemical synthesis or gene recombination technology and DNA encoding a bispecific fusion protein of the present invention into a vector including an appropriate regulatory sequence. Vectors including the regulatory sequence may be purchased commercially or prepared.

The expression vector may further include a polynucleotide encoding a secretion signal sequence, and the secretion signal sequence induces secretion of a recombinant protein intracellularly expressed out of the cell, and it may be a tissue plasminogen activator (tPA) signal sequence, a herpes simplex virus glycoprotein Ds (HSV gDs) signal sequence, or a growth hormone signal sequence.

The non-viral expression vector for gene therapy according to an exemplary embodiment of the present invention may be a DNA vector capable of expressing the GDF11 in a host cell, and the DNA vector may be any form such as a plasmid vector, a cosmid vector, a phagemid vector, an artificial human chromosome, and the like.

Moreover, gene delivery methods used in gene therapy using DNA expression vectors as described above include electroporation, gene gun, ultrasonic microbubble therapy, magnetofection, and a method of transfection using vectors such as gold or silica nanoparticles, cationic polymers, liposomes, nanoparticles, exosomes, and the like may be used. These gene transfer methods are well known in the industry. Recently, a method of directly injecting a DNA vaccine composition into a human muscle by applying an irreversible electroporation method using a small electroporation device has been used.

The activity of TRPV1, which is one of the family of permeable non-selective cationic channels, induces Ca²⁺ uptake and is inhibited by certain antagonists such as capsazepine. In addition, it is directly activated by capsaicin, heat, low pH, bradykinin, PGE2, ATP, or the like, and this activation condition means that TRPV1 is the primary biological sensor for thermo-chemical stimulation and tissue damage. TRPV1 is reported to be present in various tissues such as brain, kidney, bronchial epithelial cells and epidermal keratinocytes, and capsaicin stimulation increases the concentration of Ca²⁺ in the cytoplasm of keratinocytes, which is inhibited by capsazepine. Although the antagonist of TRPV1 has been reported as a therapeutic agent for chronic pain, since pharmacological inhibition of TRPV1 causes high fever, the development of analgesics without high fever action has been required. Accordingly, the present inventors conducted studies on a composition for treating pain that can effectively inhibit TRPV1 and confirmed that GDF11 can be a novel antagonist for pain treatment, and thus. the present inventors developed a composition for pain treatment using the inhibition of TRPV1 activity of the present invention. The present inventors observed that recombinant GDF11 inhibits the function of the TRPV1 channel associated with a concentration-dependent manner in small primary mouse dorsal root ganglions (DRG) using intracellular calcium (Ca²⁺) imaging and total-cell patch clamping. In addition, results similar to those of DRG neurons were confirmed in the TRPV1-transfected HEK293 cell line, and therefore, the above results suggest that GDF11 of the present invention can be used as a novel pain therapeutic agent that antagonizes the TRPV1 channel in chronic pain, and it can be used as a therapeutic agent that can cure various mediated diseases related to TRPV1 activity.

According to another aspect of the present invention, provided is a method for treating TRPV1-mediated disease, including a step of administering a composition comprising growth differentiation factor 11 (GDF11) peptide including an amino acid sequence of SEQ ID NO: 1, and a polynucleotide encoding the peptide or an expression vector including the polynucleotide to a patient with TRPV1-mediated disease.

Since the expression vector or TRPV1-mediated disease is the same as the concept used for the pharmaceutical composition, the description thereof is substituted with the above description.

### [Advantageous Effects]

Since the composition for treating pain using inhibition of TRPV1 activity of the present invention exhibits an excellent effect of suppressing neuropathic pain caused by spinal nerve damage, it can be used as a novel pain therapeutic agent for various pain conditions and diseases related to the TRPV1 channel such as arthritis and diabetic peripheral neuropathy. In addition, since it effectively inhibits TRPV1 activity, it can be utilized as a therapeutic agent for treating various TRPV1-mediated diseases. Certainly, the scope of the present invention is not limited by these effects.

### [Description of Drawings]

FIG. 1 is a schematic diagram schematically illustrating methods for (a) evaluating the inhibition of thermal hyperalgesia and (b) evaluating the inhibition of TRPV1 ion channel activity according to the GDF 11 treatment of the present invention by preparing a mouse chronic pain model.
FIG. 2a is a graph analyzing the delay time of avoidance response due to radiant heat stimulation according to injection of GDF11 of the present invention into a mouse model of spinal nerve transection.
FIG. 2b is a graph analyzing the delay time of avoidance response due to radiant heat stimulation according to injection of GDF11 (0.1 mg/kg) of the present invention into a mouse model of chemotherapy-induced peripheral neuropathy.
FIG. 2C is a graph analyzing the delay time of avoidance response due to radiant heat stimulation according to injection of GDF11 (0.2 mg/kg) of the present invention into a mouse model of chemotherapy-induced peripheral neuropathy.
FIG. 3a is a graph showing the calcium influx curve by treatment with GDF11 of the present invention and capsaicin in mouse sensory neurons.
FIG. 3b is a graph comparing the ratios of calcium inflow amounts by concentration compared to the control group by treatment with GDF11 of the present invention and capsaicin in mouse sensory neurons.
FIG. 4a is a graph showing the calcium influx curve by treatment with GDF 11 of the present invention and capsaicin in human embryonic kidney 293 (HEK293) cells transformed using a pcDNA-TRPV1 vector.
FIG. 4b is a graph analyzing the ratios of calcium inflow amounts by concentration compared to the control group by treatment with GDF11 of the present invention and capsaicin in human embryonic kidney 293 (HEK293) cells transformed using a pcDNA-TRPV1 vector.
FIG. 5a is a graph showing an inward current curve by treatment with GDF11 of the present invention and capsaicin in mouse sensory neurons.
FIG. 5b is a graph analyzing the ratios of the inward current size by concentration compared to the control group by treatment with GDF11 of the present invention and capsaicin in mouse sensory neurons.
FIG. 6a is a graph showing a TRPV1 inward current curve by treatment with GDF11 of the present invention and capsaicin in HEK293 cells transformed using a pcDNA-TRPV1 vector.

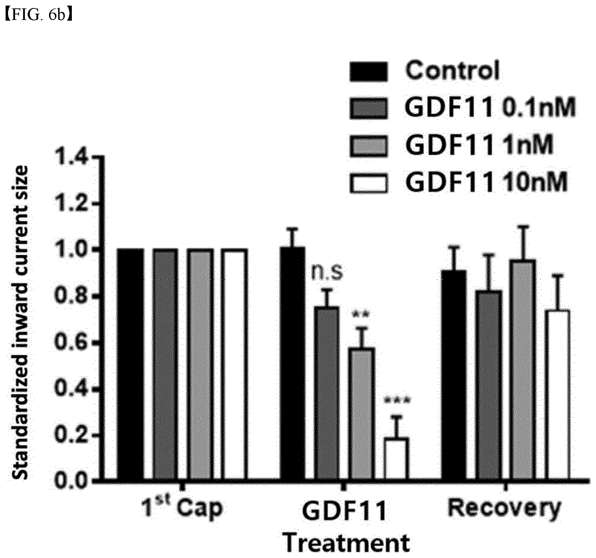

FIG. 6b is a graph analyzing the ratios of the inward current size by concentration compared to the control group by treatment with GDF11 of the present invention and capsaicin in HEK293 cells transformed using a pcDNA-TRPV1 vector.
FIG. 7 shows changes in body temperature after administration of GDF11 of the present invention to a mouse model of chemotherapy-induced peripheral neuropathy. Even when GDF11 was administered, it could be confirmed that there was no sudden change in the body temperature of the mouse.
FIG. 8 shows that an adeno-associated virus vector AAV-GDF11 expressing GDF11 of the present invention stably expresses GDF11 in mouse sensory neurons (L4 DRG or L5 DRG).
FIG. 9 is a graph analyzing the delay time of avoidance response due to radiant heat stimulation according to administration of an adeno-associated virus vector AAV-GDF11 expressing GDF11 of the present invention to a neuropathic pain mouse model.

### [Best Modes for the Invention]

### General method

### Preparation of a mouse model of spinal nerve transection of neurogenic pain

The present inventors prepared an animal model of spinal nerve transection (SNT). Specifically, 6-week-old C57BL/6 male mice were subjected to an acclimatization period for 1 week in an animal laboratory breeding environment, and then radiant heat stimulation was applied to the right hind sole using infrared heat stimulation equipment for once, and the time until avoidance response was measured to establish a reference value. Afterwards, the experimental group was randomly separated (n = 5-6 mice/group), and surgery for spinal nerve transection was performed. First, pentobarbital sodium (60 mg/kg) was injected intraperitoneally and the skin of the mouse and the like was cut under an anesthetized state to cut the 5^{th} lumbar nerve (L5 spinal nerve), and the cut muscle and skin were sutured with surgical staples.

### Construction of an animal model for peripheral neuropathy

The present inventors prepared an animal model of chemotherapy-induced peripheral neuropathy (CIPN).

Specifically, 6-week-old C57BL/6 male mice were subjected to an acclimatization period for 1 week in an animal laboratory breeding environment. 10 mg of paclitaxel (Sigma Aldrich, USA) was dissolved in 1.66 ml of a solution in which Cremophor EL and absolute ethanol were mixed at a ratio of 1:1 to make a stock at 6 mg/mL, and it was divided by 100 µL to place in microtubes and stored at -20°C. Immediately before administration, after dissolving a paclitaxel stock, it was diluted by 1/30 in saline and administered intraperitoneally with a dose of 2 mg/kg (a cumulative dosage of 10 mg/kg) once a day for 5 days to induce chemotherapy-induced pain. Heat nociceptive response was evaluated 7 days after performing the last administration, and subjects with an avoidance response time of less than 6 seconds to radiant heat stimulation were considered to have developed chemotherapy-induced pain.

### Measurement of thermal hyperalgesia

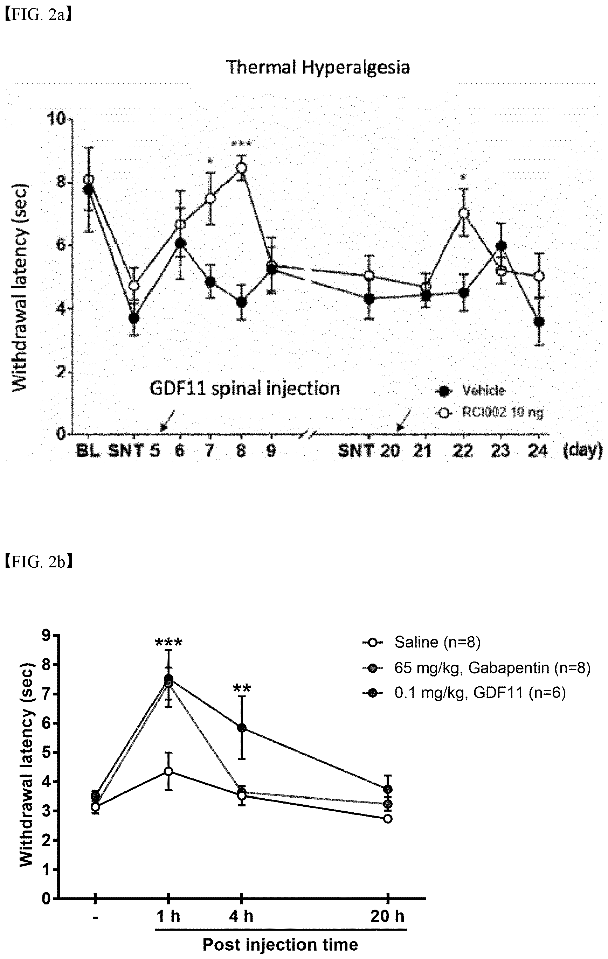

An acrylic chamber with an open bottom was placed on a glass plate of thermal pain measurement equipment (plantar test analgesia meters), and a mouse was placed in the chamber for 2 hours for 3 days to undergo a stabilization process. Before making a mouse model of spinal nerve transection, the basic pain response value against radiant heat stimulation was measured. The infrared intensity was set to 25 or 30 to obtain a basic pain avoidance response time of 8 seconds to 10 seconds. Radiant heat stimulation was applied to the right hind sole of the mouse three times at an interval of at least 1 minute or more per subject, and the avoidance response time was measured to present the average value as a heat pain response value. When evaluating the thermal pain response, the duration of the thermal stimulation was limited to 20 seconds in order to prevent thermal damage to the plantar tissue due to radiant heat stimulation. After measuring the basic pain response value, the rate of change of the response value of each subject was calculated based on the average value of all subjects, and subjects within a range of the total average value ± 50% were selected. A neuropathic pain model was constructed using only the selected subjects, and the thermal hyperalgesia response was measured on the 5^{th} day after nerve injury under the above conditions. When measuring the thermal pain response, the experimental animals were placed in an acrylic chamber for 2 hours each time, stabilized, and then subjected to radiant heat stimulation, and the thermal pain response was measured between 4 pm and 6 pm. Afterwards, the neuropathic pain animal model was arbitrarily divided into two groups and human recombinant GDF11 or saline was administered intrathecally, and then the analgesic effect of the thermal pain response was evaluated (FIG. 2a). For the experimental group, a researcher in the dark-blind state measured the thermal pain response, and after the planned thermal pain response evaluation test was completed, the results were analyzed using the information of the drug administration group to perform statistical processing.

### Administration of drug in the spinal cavity

The drug was administered directly into the spinal cord using a syringe under isoflurane respiratory anesthesia. A 30-gauge needle was connected to a Hamilton syringe, filled with GDF11 or saline, and injected into the space between the 5^{th} and 6^{th} vertebrae, and GDF11 was administered at a concentration of 10 ng/5 µL.

### Culture of mouse sensory neurons

The present inventors first cultured sensory neurons from the dorsal root ganglion (DRG) in mice. Specifically, 6 to 9-week-old C57BL/6 mice were anesthetized with isoflurane for 5 minutes, sterilized with 70% ethanol, and the back was incised. Afterwards, spinal cord DRGs of all sites were dissected and placed in a solution in which 10X HBSS and 10 mM HEPES were mixed (Wood, et al., J Neurosi. 8, pp 3208-3220, 1988). Afterwards, the dorsal root ganglion was cultured in a solution in which collagenase A at 0.2 mg/mL and dispase II at 3 mg/mL were mixed, washed once with a DMEM medium including 10% FBS, and crushed using a Pasteur pipette. Subsequently, an average of 100 to 300 cells were dispensed on a coverslip coated with poly-D-lysine, and after 1-hour incubation, it was cultured by adding 2 mL of a neurobasal medium including 10% FBS, 1% penicillin/streptomycin, and a 1X B27 supplement.

### Intracellular Ca²⁺ influx experiment

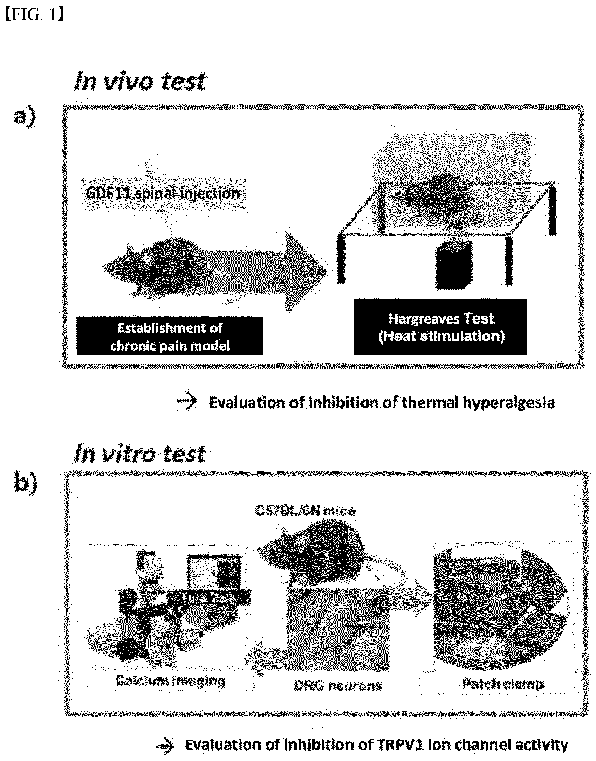

For the calcium influx measurement experiment, the primary cultured sensory neurons were exposed to a DMEM medium including 2 µM Fura-2AM for 40 minutes, and then the degree of influx of calcium ions into the cells was measured through the F340/F380 ratio, and for F340/F380 which was increased by 200 nM capsaicin, the rate of inhibition of calcium inflow by GDF11 pre-treatment was measured. Specifically, the measurement of the intracellular calcium ion concentration was performed using a microscope equipped with an imaging camera and a fluorescence measuring device connected thereto. The ratio of fluorescence wavelength (F340/F380) that appears during fluorescence measurement reflects the calcium ion concentration in the cell. The drug-containing perfusate was perfused at a rate of 2 mL/min to 3 mL/min. The composition of the perfusate was 140 mM NaCl, 5 mM KC1, 1 mM CaCl₂, 1 mM MgCl₂, 10 mM glucose, and 10 mM HEPES, and the pH was adjusted to 7.4 by adding NaOH (FIG. 1b).

### Single cell patch clamp experiment

Through the patch clamp experiment, the capsaicin-induced inward current suppression rate by GDF11 was measured. The composition of the solution in the patch pipette was 126 mM K-gluconate, 10 mM NaCl, 1 mM MgCl₂, 0.1 mM Na₂GTP, 2 mM Na₂ATP, 10 mM HEPES, 10 mM EGTA, and the pH was adjusted to 7.4 by adding KOH. The composition of the extracellular perfusate was 140 mM NaCl, 2 mM EGTA, 1 MgCl₂, 10 mM glucose, 5 mM KCl, and 10 mM HEPES, and the pH was adjusted to 7.4 by adding NaOH. The extracellular perfusate including the drug was perfused at a rate of 2 mL/min to 3 mL/min by gravity, and capsaicin and GDF11 were perfused in nociceptive neurons through the extracellular perfusate to measure the suppression rate of capsaicin-induced inward current by GDF11.

### Transformation of pcDNA TRPV1 expression vector into HEK293 cells

The present inventors simultaneously transformed the TRPV1 gene and the green fluorescence protein (GFP) gene into HEK293 cells, and then measured calcium influx and inward current through the above-described intracellular Ca²⁺ influx measurement experiment and single cell patch clamp experiment. Specifically, by using Lipofectamine™ 2000 (Invitrogen, USA), the pcDNA TRPV1 expression vector was transformed using the following method. First, after removing the medium of HEK293 cells cultured in a 35 mm cell culture dish, only a DMEM medium was added and it was wet cultured at 37°C in 5% CO₂, and pcDNA-TRPV1 (1 µg) and Lipofectamine (10 µL) were dissolved in each DMEM medium. After waiting for 5 minutes, the two media were mixed and waited for another 20 minutes. Afterwards, the DMEM medium of a dish to which HEK293 cells were attached was removed, and the medium containing the previously prepared pcDNA was dispensed, followed by wet culture at 5% CO₂ and 37°C for 4 hours. It was changed with a DMEM medium containing 10% FBS and 1% penicillin/streptomycin, and after culturing for 12 hours, calcium imaging or the patch clamp experiment was performed within 24 hours.

### Preparation of viral vectors

The present inventors attempted to prepare a viral vector containing the GDF11 gene.

Specifically, the AAV5 serotype and CMV promoter were selected in order to induce the constant expression of GDF11 in the cells that infected or infection to neurons. In addition, the internal ribosome entry site (IRES) and enhanced green fluorescence protein (eGFP) nucleotide sequences were added after the GDF11 nucleotide sequence such that GDF11 and GFP proteins could be expressed independently of each other in the infected cells. Distribution of the infected cells and expression of GDF11 in the tissues of experimental animals can be evaluated. After viral vectors were produced by loading the GDF11 gene in the AAV5 vector, the insertion of the normal GDF11 gene was verified through nucleotide sequencing, and the titer of the tested GDF11-expressing virus was 1.22 × 10¹³ GC/mL.

### Tissue immunostaining

The present inventors performed tissue immunostaining in order to confirm the expression of GDF11 in sensory neurons after intrathecal injection of a viral vector containing the gene.

Specifically, pentobarbital sodium (60 mg/kg) was injected intraperitoneally into experimental animals and anesthetized. A 30 mL syringe was filled with 30 mL of a 0.9% NaCl solution, and the right atrium side of the heart was incised, and the syringe was pierced into the left ventricle to remove blood. Subsequently, 30 mL of a 10% formalin solution was administered to the left ventricle to perform fixation, and then L4 and L5 DRG tissues were sampled. The removed DRG tissue was immersed in a 10% formalin solution for 16 hours to perform additional fixation, and then the tissue was transferred to a 30% sucrose solution and dehydration was performed for two days. Afterwards, the DRG tissue was frozen at -20°C, and then a sectioned tissue was made with a thickness of 12 µm. Tubulin antibody (Santa Cruz, USA) was diluted by 1/100 in a 0.1 M PBS solution and treated on DRG tissue sections for 24 hours, and after washing with 0.1 M PBS, fluorescence images were captured using a confocal microscope.

### Experimental statistics

Statistical analysis was performed using the prism (Graphpad, version 501) software. Specifically, after confirming whether it was the normal distribution through a normality test, parametric or nonparametric tests were used. For the parametric and nonparametric tests, t-test was performed for two or more groups, and for three or more groups, it was analyzed by a one-way ANOVA test or two-way ANOVA test. Error bars were visualized by the standard error of measurement (SEM) and if the experimental result was significant, it was shown as ^{∗} or # [1 for P < 0.05, 2 for P < 0.005, and 3 for P < 0.001].

### [Example 1]

### Evaluation of thermal hyperalgesia

### <1-1> Mouse model of chronic pain by spinal nerve transection

The present inventors prepared an experimental model of mice with chronic pain by spinal nerve transection (SNT), and after administering GDF11 (10 ng) into the spinal cord, the thermal pain response was evaluated using the Hargreaves equipment. As a result, compared to the control group, it was found that the delay time of the hind sole avoidance response was increased by suppressing a phenomenon of thermal hyperalgesia according to the administration of GDF11 of the present invention to the chronic pain mouse model (FIG. 2a).

### <1-2> Mouse model of peripheral neuropathy

The present inventors prepared a mouse experimental model of chemotherapy-induced peripheral neuropathy (CIPN), and after intraperitoneal administration of 0.1 mg/kg or 0.2 mg/kg of GDF11, the thermal pain response was evaluated using the Hargreaves equipment.

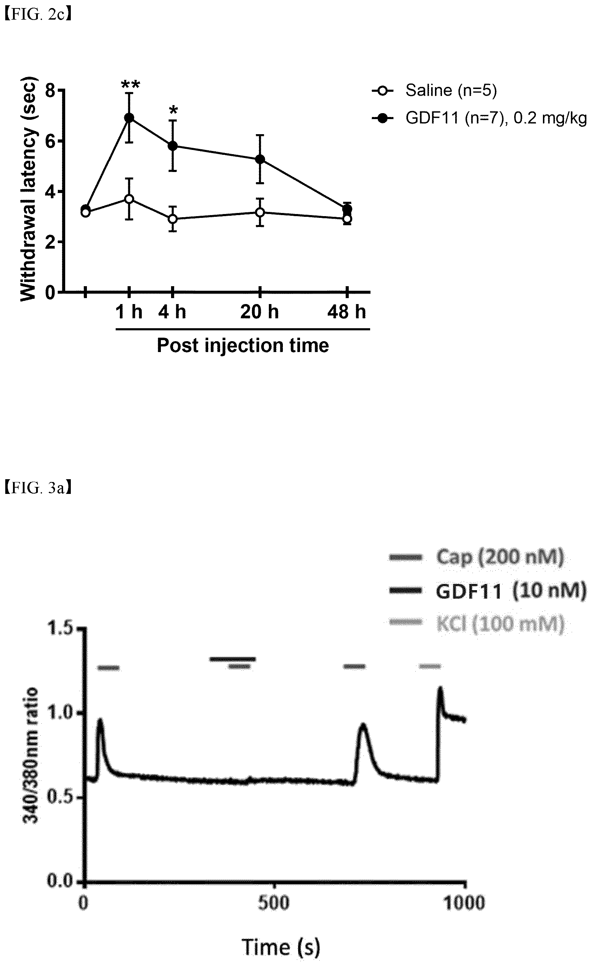

As a result, as shown in [FIG. 2b] and [FIG. 2c], it was confirmed that the delay time of avoidance response was increased by suppressing the phenomenon of thermal hyperalgesia according to the administration of GDF11.In particular, it was found that GDF11 inhibited a phenomenon of thermal hyperalgesia for a longer period of time at an amount 650 times less than gabapentin (Sigma Aldrich, USA), which is one of the representative pain therapeutic agents (FIG. 2b).

### [Example 2]

### Analysis of calcium influx amount in mouse sensory neurons and HEK293 cells

The amount of calcium influx due to the treatment of GDF11 (10 nM) of the present invention and capsaicin (200 nM) in primary cultured mouse sensory neurons and HEK293 cells transformed using the pcDNA-TRPV1 vector, and the amount of calcium inflow according to the treatment by GDF11 concentrations (0.1 nM, 1 nM. or 10 nM) were compared and analyzed.

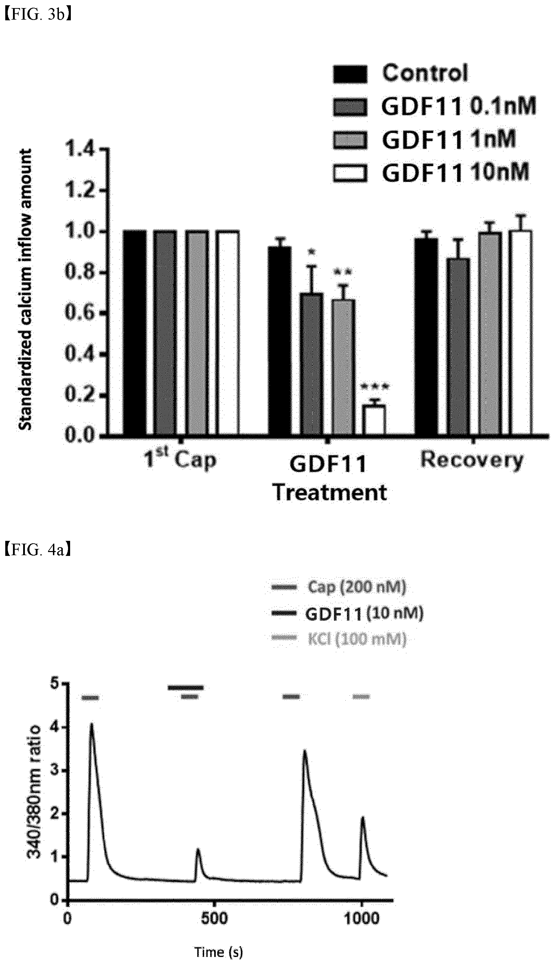

As a result, compared to the control group which was not treated with GDF11, the treatment of GDF11 of the present invention was found to decrease a TRPV1-mediated Ca²⁺ influx (Ca2⁺ transient) phenomenon in a concentration-dependent manner (FIGS. 3a, 3b 4a, and 4b). The degree of calcium influx was standardized and shown based on the size of the first calcium response by capsaicin.

### [Example 3]

### Analysis of the size of inward current in mouse sensory neurons and HEK293 cells

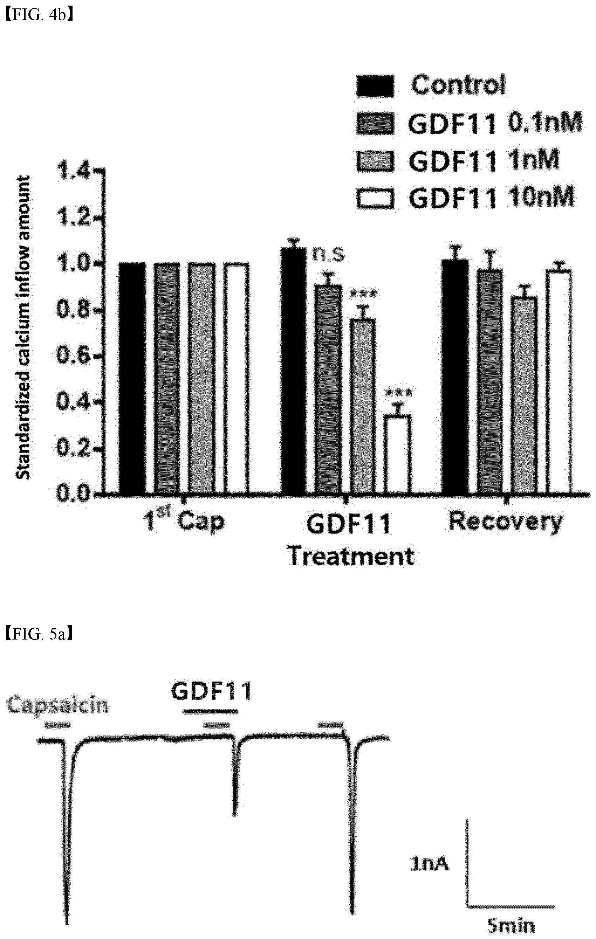

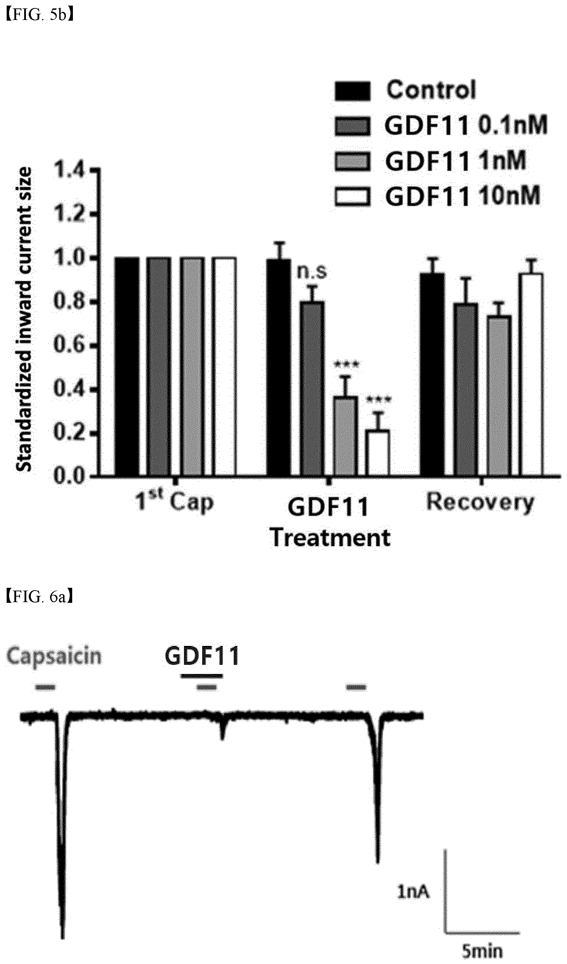

The size of inward current due to the treatment of GDF11 (10 nM) of the present invention and capsaicin (200 nM) in primary cultured mouse sensory neurons and HEK293 cells transformed using the pcDNA-TRPV1 vector, and the size of inward current according to the treatment by GDF11 concentrations (0.1 nM, 1 nM. or 10 nM) were compared and analyzed. As a result, compared to the control group which was not treated with GDF11, the GDF11 of the present invention was found to decrease a TRPV1-mediated inward current which was induced by capsaicin in a concentration-dependent manner (FIGS. 5a, 5b 6a, and 6b) in the mouse sensory neurons. The measurement of inward current was standardized and shown based on the size of the first response by capsaicin.

The results of Examples 1 to 3 above suggest that since the treatment of GDF11 effectively inhibits TRPV1 ion channel activity at a low concentration, it can be utilized as an effective antagonist of TRPV1.

### [Example 4]

### Confirmation of side effects of GDF11

Existing TRPV1 antagonists are known to have side effects such as abnormal fever or hypothermia under administration conditions showing analgesic efficacy, and thus, the present applicants attempted to confirm whether the side effects occurred due to the administration of GDF11.

Specifically, BCTC (30 mg/kg) or GDF11 (0.1 mg/kg or 0.2 mg/kg) at a concentration that suppresses TRPV1 activity by capsaicin to a similar degree in model mice having chemotherapy-induced pain, in which the skin was exposed by shaving the hair on a portion of the back side, was administered intraperitoneally, and then the temperature of the exposed skin was measured by time intervals using a thermal imaging camera.

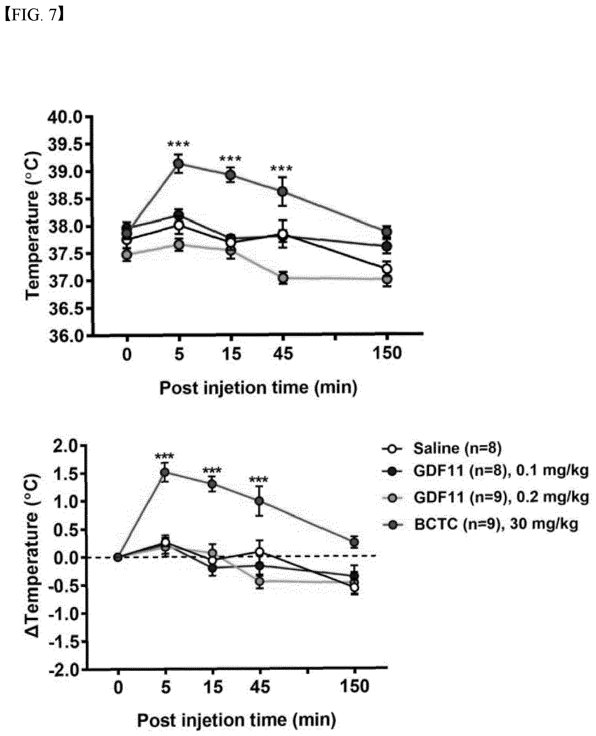

As a result, as shown in [FIG. 7], even if GDF11 was administered, no significant change occurred in the body temperature of the mice, and it could be confirmed that the GDF11 administration did not cause side effects of abnormal fever or hypothermia. On the other hand, when BCTC, which is a representative TRPV1 antagonist, was administered intraperitoneally at a dose of 30 mg/kg, a side effect of abnormal fever appeared.

### [Example 5]

### Confirmation of GDF11 expression of viral vector

The present inventors attempted to determine whether GDF11 expression *in vivo* is induced when the prepared viral vector is administered.

Specifically, 5 µL of the GDF11 viral vector at a concentration of 1.22×10¹¹ GC/mL was injected into the spinal cord in the neuropathic pain model caused by peripheral spinal nerve injury. After 5 weeks, mice were sacrificed to sample L4 and L5 DRG tissues, and lyophilized sections were prepared. Tissues were immunostained with the Tubulin antibody, and the GFP fluorescence signal was regarded as the expression level of GDF11, and imaging was performed under a confocal microscope.

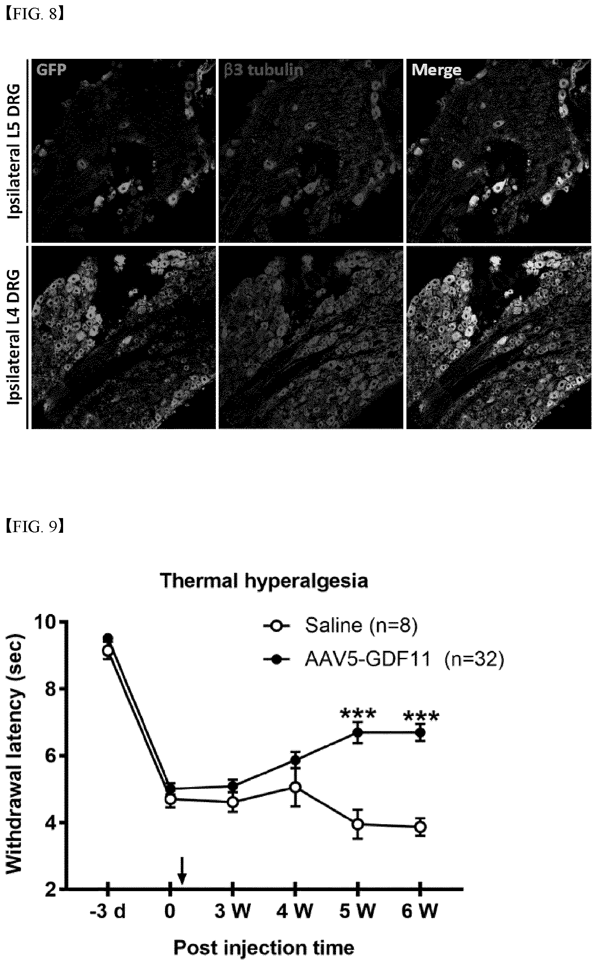

As a result, it was confirmed that GDF11 was expressed in L4 or L5 DRG neurons of mice as shown in [FIG. 8].

### [Example 6]

### Confirmation of the pain relief effect of viral vectors

The present inventors attempted to determine whether the prepared viral vector AAV5-GDF11 exhibits an analgesic effect in an animal model of neuropathic pain.

Specifically, a neuropathic pain model was prepared by cutting the peripheral spinal nerve after measuring the basic pain response value before preparing the pain model. After 3 days of surgery for the pain model, the thermal pain response was evaluated, and subjects whose avoidance response threshold fell below 6 seconds were considered as a pain model. In this pain model, 5 µL of the GDF11 viral vector at a concentration of 1.22 × 10¹¹ GC/mL was injected into the spinal cord, and the thermal pain reaction was evaluated once a week using the Hargreaves equipment.

As a result, as shown in [FIG. 9], it was confirmed that by administering AAV5-GDF11, the delay time of avoidance response was increased by suppressing the phenomenon of thermal hyperalgesia.

Comprehensively, since GDF11 of the present invention, which is a composition for treating pain using inhibition of TRPV1 activity, has been shown to quickly and effectively inhibit TRPV1 channels, it can be utilized as a novel therapeutic agent for various pain conditions and diseases related to the TRPV1 channel, such as arthritis and diabetic peripheral neuropathy.

The present invention has been described with reference to the above-described exemplary embodiments, but these are merely exemplary, and those of ordinary skill in the art will understand that various modifications and equivalent other exemplary embodiments are possible therefrom. Therefore, the true technical protection scope of the present invention should be determined by the technical spirit of the appended claims.

### [Industrial Applicability]

Since the composition for treating pain using the inhibition of TRPV1 activity of the present invention exhibits an excellent effect of suppressing neuropathic pain caused by spinal nerve damage, it can be used as a novel pain therapeutic agent for various pain conditions and diseases related to the TRPV1 channel such as arthritis and diabetic peripheral neuropathy. In addition, since it effectively inhibits TRPV1 activity, it can be utilized as a therapeutic agent for treating various TRPV1-mediated diseases, and thus, it has high industrial applicability.

### [Sequence Listing Free Text]

SEQ ID NO: 1 shows an amino acid sequence of a growth differentiation factor 11 (GDF11) mature peptide.
SEQ ID NO: 2 shows a full-length amino acid sequence of human GDF11 peptide.
SEQ ID NO: 3 shows an amino acid sequence of mouse GDF11.
SEQ ID NO: 4 shows an amino acid sequence of chimpanzee GDF11.
SEQ ID NO: 5 shows an amino acid sequence of gorilla GDF11.
SEQ ID NO: 6 shows an amino acid sequence of golden snub-nosed monkey GDF11.
SEQ ID NO: 7 shows an amino acid sequence of Northern white-cheeked gibbon GDF11.
SEQ ID NO: 8 shows an amino acid sequence of Bengal monkey GDF11. SEQ ID NO: 9 shows an amino acid sequence of bonobo GDF11.
SEQ ID NO: 10 shows an amino acid sequence of pig GDF11.
SEQ ID NO: 11 shows a polynucleotide sequence encoding the GDF11 peptide (SEQ ID NO: 1).

## Claims

1. A pharmaceutical composition for treating TRPV1-mediated disease, comprising:
a growth differentiation factor 11 (GDF11) peptide comprising an amino acid sequence of SEQ ID NO: 1, a polynucleotide encoding the peptide or an expression vector comprising the polynucleotide as an active ingredient.

2. The pharmaceutical composition of claim 1, wherein the expression vector is a viral vector or a non-viral vector.

3. The pharmaceutical composition of claim 2, wherein the viral vector is an adeno-associated virus (AAV) vector, an adenovirus vector, an alphavirus vector, a herpes simplex virus vector, a vaccinia vector, a Sendai virus vector, a flavivirus vector, a rhabdovirus vector, a retrovirus vector, or a lentivirus vector.

4. The pharmaceutical composition of claim 3, wherein the serotype of the adeno-associated virus (AAV) vector is AAV1, AAV2, AAV3, AAV4, AAV5, AAV6, AAV7, AAV8, AAV9, AAV10, AAV11, AAV12, AAV13, AAV14, AAV15, or AAV16.

5. The pharmaceutical composition of claim 2, wherein the non-viral vector is a DNA vector, a nanoparticle, a cationic polymer, an exosome, an extracellular vesicle, or a liposome.

6. The pharmaceutical composition of claim 5, wherein the DNA vector is a plasmid vector, a cosmid vector, a phagemid vector, or an artificial human chromosome.

7. The pharmaceutical composition of claim 1, wherein the TRPV1-mediated disease is selected from the group consisting of pain, hypertension, stroke, myocardial ischemia, urinary incontinence, urinary bladder hypersensitiveness, irritable bowel syndrome, fecal urgency, stomach-duodenal ulcer, gastro-esophageal reflux disease (GERD), Crohn's disease, haemorrhoid, asthma, chronic obstructive pulmonary disease, pruritus, psoriasis, hearing loss, tinnitus, cough, hypertrichosis, and alopecia.

8. The pharmaceutical composition of claim 7, wherein the pain is nociceptive pain, psychogenic pain, inflammatory pain, or pathological pain.

9. The pharmaceutical composition of claim 8, wherein the pathological pain is selected from the group consisting of neuropathic pain, cancer pain, chemotherapy-induced pain, postoperative pain, trigeminal neuralgia pain, idiopathic pain, diabetic neuropathic pain, migraine, arthralgia, and neuralgia.

10. A method for treating TRPV1-mediated disease, comprising administering a composition comprising growth differentiation factor 11 (GDF11) peptide comprising an amino acid sequence of SEQ ID NO: 1, a polynucleotide encoding the peptide or an expression vector comprising the polynucleotide to a patient with TRPV1-mediated disease.
